# EUROPEAN PATENT SPECIFICATION

(11) **EP 3 034 019 B1**
(45) Date of publication and mention of the grant of the patent: **28.06.2017**
(21) Application number: 14197931.0
(22) Date of filing: 15.12.2014
(51) Int. Cl.: A61B 17/29, A61B 17/00

(54) **Medical instrument for grasping tissue**
Medizinisches Instrument zum Greifen von Gewebe
Instrument médical servant à saisir des tissus

(43) Date of publication of application: 22.06.2016
(73) Proprietor: University Of Dundee, Dundee DD1 4HN (GB)
(72) Inventor: Wang, Zhigang, Dundee DD2 1FD (GB); Cuschieri, Alfred, St. Andrews Fife KY16 9TY (GB)
(74) Representative: Witte, Weller & Partner Patentanwälte mbB

(56) References cited:
- WO-A1-2014/155279
- DE-C1- 10 024 397
- US-A1- 2007 135 685
- US-A1- 2008 297 287
- US-A1- 2010 063 475
- US-A1- 2011 301 602
- US-A1- 2012 101 488
- US-A1- 2013 197 516

## Description

The invention relates to a medical instrument for grasping tissue, comprising a shaft, a working element arrangement arranged at the distal end of the shaft and having a first working element and a second working element configured to cooperate with one another for grasping tissue, wherein at least the first working element is movable, so that the working element arrangement can be closed and opened, further comprising a magnet arrangement coupled to the working element arrangement for closing and opening the working element arrangement via attractive and repulsive magnetic forces, respectively, according to the preamble of claim 1.

A medical instrument of the type mentioned at the outset is known fromUS 2011/0301602 A1.

In surgical operations on patients, it is often necessary to manipulate tissues or organs during the surgical procedures. Such manipulations can consist in putting aside an organ or tissue in order to obtain an access to deeper lying tissue or organs on which the surgical task is to be performed, or in retracting tissue or an organ for a subsequent dissection, for example.

Surgical tasks necessitating the manipulation of tissue or organs can arise in minimally invasive surgeries as well as in open surgeries.

Conventional instruments which are used for the purpose of manipulating tissue comprise a working element arrangement also referred to as jaw arrangement, having at least two working elements, one or both of which are movable with respect to the shaft of the instrument. Thus, the working element arrangement can be closed and opened, wherein the working element arrangement is opened to engage the tissue, and by exerting a closing force onto the working element arrangement, the tissue is retained between the working elements. In these conventional medical instruments, closing and opening of the working element arrangement is actuated by operating a handle arranged at the proximal end of the shaft and having at least one movable grip part, which is connected through a force transmission element to the at least one movable working elements. For example, the handle of the instrument is configured as a scissors grip handle. The working elements have grasping surfaces opposite to each other to grasp the tissue. In order to enhance the grip of the grasping surfaces of the working element arrangement, the grasping surfaces have sharp features like teeth..

A medical instrument known from US 2012/0101488 A1 comprises, in addition to the afore-mentioned conventional force transmission from the handle to the working element arrangement a magnet arrangement, wherein the one working element comprises one magnet, and the other working element also comprises one magnet. The magnetic motive force between the magnets of the working elements causes them to attract each other to thereby reduce the external force required to transition the first and second working elements to the closed position. In addition, the magnets may be configured to repell each other to thereby aid in opening the working element arrangement. In an embodiment, one of the working elements has a magnet which is rotatable. The magnetic polarity of the magnets of the known instrument is perpendicular to the opening and closing movement of the working element arrangement, the rotation axis being perpendicular to the longitudinal axis of the working element arrangement.

A disadvantage of this known medical instrument is that the magnet arrangement only aids in closing and opening the working element arrangement. The known medical instrument and the conventional medical instruments mentioned before have the following further disadvantages. The surface profiling of the working elements with teeth, hooks and the like results in a very high local pressure on the grasped tissue, due to the small contact area, with potential risk of causing tissue damage. Further, since the force for closing the working element arrangement and thus the grasping force is exerted by the hand (hand force) of the operating surgeon, it is difficult to properly dose the closing force. A closing force which is too high can damage the grasped tissue, while a closing force which is too low leads to an insufficient retention of the tissue between the working elements, in particular for the purpose of retracting the tissue.

US 2011/0301602 A1 discloses an endoscopic forceps comprising an end effector assembly operatively connected to a distal end of the shaft and including a pair of first and second jaw members that are pivotably coupled to one another and movable relative to one another. A magnetic actuation mechanism is operably coupled to one or both of the first and second jaw members and configured to generate opposing magnetic fields on each of the first jaw and second jaw members to actuate the first and second jaw members between the first and second configurations.

Further, US 2007/0135685 A1 discloses a medical instrument, which comprises a shaft and one or more working elements arranged at a distal end portion of the shaft. The working element comprises one or more magnetically acting elements generating a magnetic field for manipulating the tissue. This medical instrument requires that the tissue to be manipulated by magnetic forces is treated with a magnetically acting substance in advance, in order to be able to be grasped by the instrument through magnetic forces. Tissue magnetization can be done by injecting magnetic materials into the tissue, or by applying magnetic glue onto the surface of the tissue. Besides an additional expenditure of time for preparing the tissue in this way, a further disadvantage is that it is difficult to properly remove the injected magnetic articles or the magnetic glue after the surgery.

It is therefore an object of the present invention to improve a medical instrument for grasping tissue mentioned at the outset such that the instrument provides a controllable force for grasping tissue in an atraumatic way. Further, the instrument should preferably provide a force for grasping tissue, which is independent from the hand force of the user of the instrument, and which does not require to magnetize the tissue to be grasped.

According to an aspect of the present invention, the object underlying the invention is achieved in that at least one third magnet is arranged between the first and second working elements, the third magnet being rotatable about a rotation axis.

In this configuration, the working element arrangement can be designed in "chop-stick" fashion with at least two working elements, co-acting with one another for grasping tissue. Switching between a magnetic attractive force for closing the working element arrangement and a magnetic repulsive force for opening the working element arrangement is accomplished by the rotatable third magnet positioned in-between the first and second magnets. As in the first aspect of the invention, the magnitude of the magnetic force acting between the third magnet and the first and second magnets is continuously adjustable by a rotation of the third magnet.

Further, the magnetic attractive force preferably is the sole force for closing the working element arrangement, and/or the magnetic repulsive force preferably is the sole force for opening the working element arrangement.

Preferably, the magnetic polarities of the first and second magnets are parallel with respect to one another and parallel to the closing and opening movement of the first and second working elements.

Further, the rotation axis preferably is parallel to the longitudinal axis of the shaft, and the third magnet preferably has a magnetic polarity perpendicular to the rotation axis.

A simple actuating mechanism for rotating the third magnet is obtained in that the third magnet is connected to a rotatable actuating rod.

The first and second working elements are preferably configured as elongate fingers each having a distal grasping area, and wherein the first and second magnets are arranged in a proximal non-grasping area of the elongate fingers.

In this configuration, the magnetic mechanism for opening and closing the working element arrangement is spaced apart from the gripping area for grasping the tissue. In this way, the third rotatable magnet does not interfere with the grasping areas of the first and second working elements.

In a further preferred refinement, the instrument has at least one third working element having a further magnet, wherein the at least three working elements are circumferentially distributed about the third magnet.

This configuration has the advantage that the working element arrangement provides a human hand-like prehensile grasping of tissue, and the risk of tissue slip is further reduced.

The magnets of the magnet arrangements according to both aspects of the invention preferably are permanent magnets, but they can also be configured as electromagnets. The use of permanent magnets has the advantage that they produce magnetic fields without a need for electric power supply.

The medical instrument of the invention is suitable for use in open surgeries, but also in minimally invasive or minimal access surgeries.

The first working element and the second working element may each have a grasping surface for grasping tissue, one of the grasping surfaces having an array of first further magnets, and the at least one other grasping surface has at least one second further magnet or an array of second further magnets for attractive interaction with the first further magnets.

This aspect additionally solves the problem of conventional medical grasping instruments having sharp features like teeth, hooks and the like, in order to prevent slipping of the grasped tissue from the working element arrangement. By using magnets, which may be embedded in the grasping surfaces of the working elements or arranged on top of the grasping surfaces, slip prevention can be achieved without sharp features. With the present aspect, a kind of magnetic force surface profiling is achieved which can replace the conventional surface profiling having sharp features at the grasping surfaces.

In refinements of this aspect, the first further magnets can be configured as magnetic strips on top of or embedded in the one grasping surface, while the grasping surface of the other working element has a single magnetic plate, preferably embedded therein.

In another preferred refinement, the array of the first further magnets has small disk magnets having the same or different sizes and distributed along the grasping surface of one of the working elements. It is also possible that each working element has an array of further magnets, preferably in form of small disk magnets, which can be embedded in the grasping surfaces of the working elements.

Further, the working elements can have a fenestration in the area of the grasping surfaces for further improving tissue retention.

Further advantages and features will become apparent from the following description and accompanying drawings.

It is to be understood that the afore-mentioned features and those features still to be explained below are not only applicable in the combinations given, but also in other combinations or in isolation without departing from the scope of the present invention.

Exemplary embodiments are shown in the drawings and will be described with reference thereto in the following. In the drawings:
- Fig. 1A: shows an example of a medical instrument for grasping tissue in a side view, wherein a working element arrangement is closed;
- Fig. 1B: shows the medical instrument in Fig. 1A, wherein the working element arrangement is open;
- Fig. 2: shows the working element arrangement of the medical instrument in Fig. 1A in an enlarged scale and in a perspective view,
- Fig. 3A: shows a longitudinal section along a line IIIA-IIIA in Fig. 2 through the working element arrangement in Fig. 2;
- Fig. 3B: shows the same longitudinal section as in Fig. 3A, wherein the working element arrangement is open;
- Fig. 4: shows a distal portion of the medical instrument in Fig. 1 grasping tissue;
- Fig. 5: shows a diagram illustrating a magnetic attractive force and a magnetic repulsive force acting between the working elements of the working element arrangement of the instrument in Fig. 1 as a function of magnet displacement;
- Fig. 6: shows a handle modified in comparison with the handle of the medical instrument shown in Figs. 1A and 1B;
- Fig- 7: shows another handle modified in comparison with the handle of the medical instrument shown in Figs. 1A and 1B;
- Fig. 8A: shows an embodiment of a working element arrangement for a medical instrument according to the invention for grasping tissue in a perspective view, wherein the working element arrangement is closed;
- Fig. 8B: shows the working element arrangement in Fig. 8A, wherein the working element arrangement is open;
- Fig. 8C: shows a magnet of the working element arrangement in Figs. 8A and 8B;
- Fig. 9A: shows another working element arrangement of a medical instrument for grasping tissue in a perspective view, wherein the working element arrangement is closed;
- Fig. 9B: shows the working element arrangement in Fig. 9A, wherein the working element arrangement is open;
- Fig. 10: shows two working elements in an open state with a magnetic surface profiling in a perspective view;
- Fig. 11: shows another two working elements with a magnetic surface profiling in a perspective view; and
- Fig. 12: shows another two working elements with a magnetic surface profiling in a perspective view.

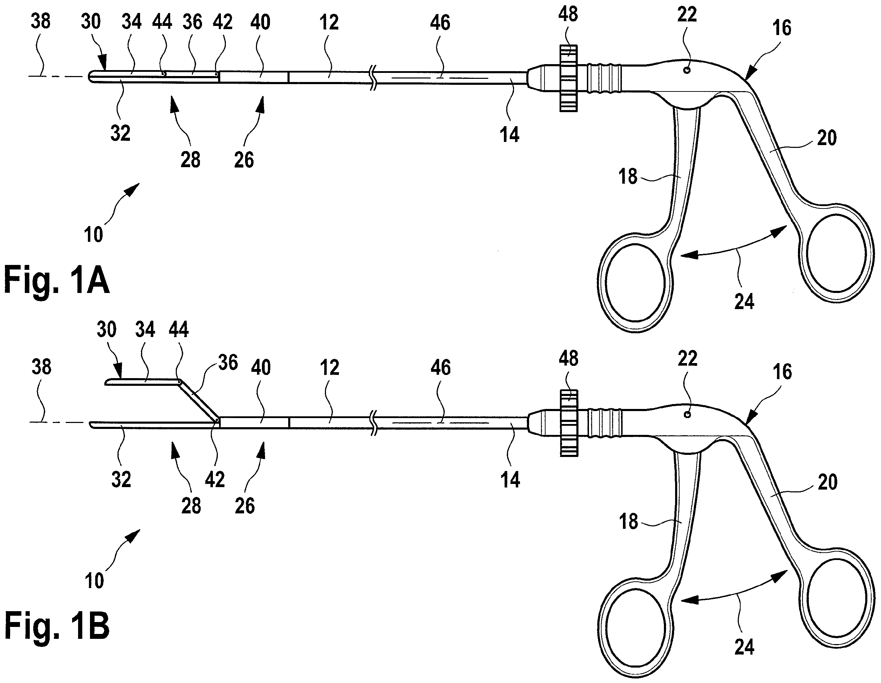

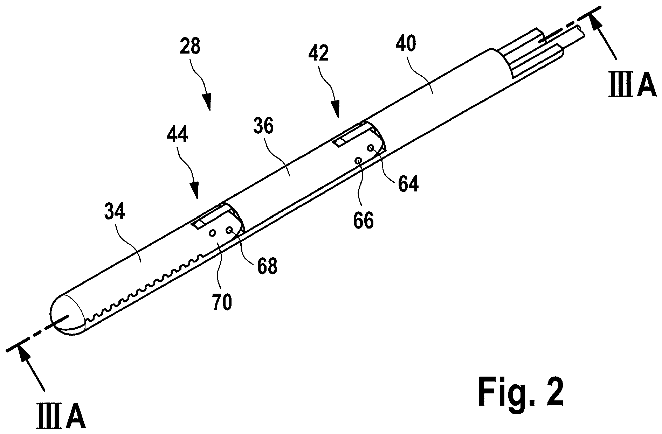

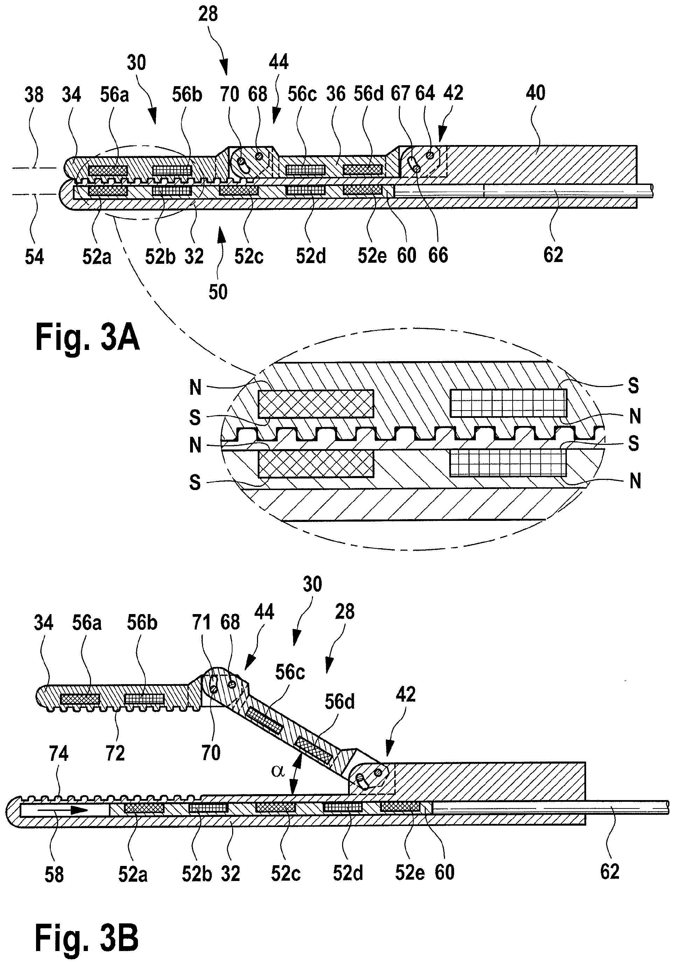

Figs. 1A and 1 B show a medical instrument for grasping tissue generally labeled with reference numeral 10. Figs. 2, 3A, 3B show further details of the instrument 10.

With reference to Figs. 1A and 1 B, the instrument 10 comprises an elongated shaft 12 of small outer diameter, e.g. 5 mm, so that the instrument 10 is suitable for minimally invasive or minimal access surgeries. The shaft 12 has a proximal end 14 at which a handle 16 is arranged having a movable grip part 18 and a stationary grip part 20. The movable grip part 18 is pivotable about a pivot axis 22 according to a double arrow 24 relative to the grip part 20.

At a distal end 26 of the shaft 12, a working element arrangement 28 is connected to the shaft 12. The working element arrangement 28 has a first working element 30 and a second working element 32. The first working element 30 is movable with respect to the shaft 12, while the second working element 32 is stationary with respect to the shaft 12. Thus, the working element arrangement 28 can be closed as shown in Fig. 1A, and can be opened as shown in Fig. 1 B. Closing and opening of the working element arrangement 28 is realized via attractive and repulsive magnetic forces, respectively, as will be described below. An attractive magnetic force is used for closing the working element arrangement 28, and a repulsive magnetic force is used for opening the working element arrangement.

As further shown in Figs. 1A and 1 B, the first working element 30 has at least two, in the present example exactly two segments 34 and 36, which are arranged in series along a longitudinal axis 38 of the first working element 30.

The working element arrangement 28 comprises a shaft connector 40 for connecting the working element arrangement 28 to the shaft 12. In the assembled state of the medical instrument 10, the shaft connector 40 forms part of the distal end 26 of the shaft 12.

The segment 36 of the working element 30 is connected to the shaft connector 40 and, thus, to the shaft 12 via a first hinge 42, and the segment 34 is connected to the segment 36 via a second hinge 44.

Further, the shaft 12 and, thus, the working element arrangement 28 are rotatable about a longitudinal axis 46 of the shaft 12, wherein rotation can be actuated by a wheel 48 arranged at the handle 16.

With reference to Fig. 2, 3A, and 3B, details of the magnetic mechanism for opening and closing the working element arrangement 28 will be described.

The mechanism for closing and opening the working element arrangement 28 comprises a magnet arrangement 50 coupled to the working element arrangement 28 for closing and opening the working element arrangement via attracting and repulsive magnetic forces, respectively. The magnet arrangement 50 has a plurality of magnets 52a, 52b, 52c, 52d, 52e arranged on the stationary working element 32. The magnets 52a-52e are arranged in series along a longitudinal axis 54 of the working element 32. The magnets 52a-52e are permanent disc-shaped magnets, but can also be of other shapes such as plates (square or rectangular, etc.).

The magnets 52a-52e have alternating magnetic polarities with respect to one another. In the enlarged cut-out in Fig. 3A, this is illustrated by magnetic poles S (magnetic south poles) and magnetic poles N (magnetic north poles). In Fig. 3A, those magnets having a magnetic polarity N-S when seen from bottom to top in the plane of drawing are shown with cross-hatchings having horizontal and vertical lines, and those magnets having a magnetic polarity S-N when seen from bottom to top in the plane of drawing are shown with cross-hatchings having lines inclined by 45° to the horizontal.

The magnetic polarities of the magnets 52a-52e are perpendicular to the longitudinal axis 54 of the working element 32 and are in the plane of the closing and opening movement of the working element arrangement 28, which plane is the plane of drawing in Figs. 3A and 3B.

The magnet arrangement 50 further has a plurality of magnets 56a, 56b, 56c, 56d arranged on the first working element 30.

The magnets 56a-56d are arranged in series along the longitudinal axis 38 of the working element 30. The magnets 56a and 56b are arranged on the segment 34, and the magnets 56c and 56d are arranged on the segment 36.

The magnets 56a, 56b, have alternating polarities with respect to one another, and the magnets 56c, 56d have alternating polarities with respect to one another.

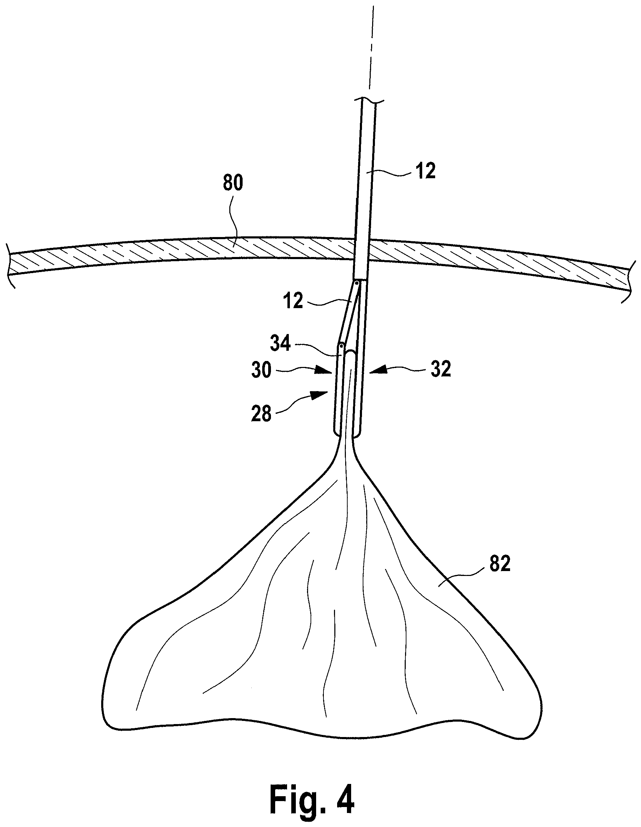

In Fig. 3A, in the closed state of the working elements 30 and 32, the magnets 52a and 56a are paired with one another with parallel polarities, which means that when seen in direction from bottom to top in the drawing, magnet 52a has a polarity S-N, and also magnet 56a has a polarity S-N, thus unlike poles face each other. Further, magnets 52b and 56b are paired with parallel polarities, as well as magnets 52d and 56c and also magnets 52e and 56d, i.e. unlike poles of these magnet pairs face each other. Thus, there is an attractive magnetic force between the magnet pairs 52a, 56a; 52b, 56b; 52d; 56c; and 52e and 56d. In this state of paired first magnets 52a, 52b, 52d, 52e and second magnets 56a, 56b, 56c, 56d, the attractive magnetic force urges the working element arrangement 28 in the closed state as shown in Fig. 3A. In the state shown in Fig. 3A, the magnetic attractive force is maximum. If there is tissue grasped between the working elements as shown in Fig. 4, the magnitude of the magnetic attractive force is somewhat smaller, because of the larger distance between the attractively paired magnets than in the state shown in Fig. 3A.

The first magnets 52a-52e are displaceable in direction of the longitudinal axis 54 of the working element 32 as shown in Fig. 3B, where the magnets 52a-52e are longitudinally displaced according to an arrow 58. The magnets 52a-52e are arranged in a magnet housing 60, which can be slidably displaced, on the working element 32.

The magnets 52a-52e are connected via the housing 60 with a push/pull rod 62 which in turn is connected with the movable grip part 18 in Figs. 1A and 1 B. By actuating the movable grip part 18 according to the arrow 24 in Fig. 1A, the housing 60 and thus the magnets 52a-52e can be longitudinally displaced in distal or proximal direction with respect to the magnets 56a-56d. A higher force is needed to displace the housing 60 in order to open the working element arrangement 28 which needs to overcome the maximum magnetic attractive force at the closed state. On the other hand, it is much easier to slide the housing 60 in order to close the working element arrangement 28, since at initial fully opened stage, the magnetic interaction force (mainly repulsive) is weaker due to a larger distance between the paired magnets, and at closer distance, where magnetic attractive force is the main force, which will actually facilitate the housing return to its initial stage, where the working element arrangement 28 is closed, by aligning the paired magnets. In this example, a pull force is used to longitudinally displace the housing 60 toward (arrow 58) the proximal direction in order to open the working element arrangement 28 (Fig. 3B). This can be manually ergonomically provided by the movable grip part 18 of the handle. Alternatively, the push/pull rod can be driven by a motor, for example a piezo-actuator or a linear motor, arranged at a proximal end of the instrument.

Fig. 3B shows a situation, where the magnets 52a-52e are displaced so far that now magnet 52a is paired with magnet 56b, and magnet 52c is paired with magnet 56c and magnet 52d is paired with magnet 56d. Since the magnets 52a and 56b have antiparallel magnetic polarities, i.e. like poles of the magnets 52a and 56b face each other, a repulsive magnetic force acts between the magnets 52a and 56b. The same holds for the magnets 52c and 56c as well as 52d and 56d. Due to the repulsive magnetic forces, the working element arrangement 28 is opened.

When the magnets 52a-52e are paired with the magnets 56a-56d as shown in Fig. 3A, the attractive magnetic force is maximum. This maximum attractive force can be lowered by displacing the magnets 52a-52e with respect to the magnets 56a-56d over a slight distance such that there is still an attractive force, but lowered in comparison to the state as shown in Fig. 3A. In this way, the magnitude of the maximum attractive magnetic force can be continuously controlled by displacing the magnets 52a-52e. Thus, the magnitude of the magnetic attractive force can be adjusted by moving the movable grip part 18 in order to displace the magnets 52a-52e.

In the present example, the magnets 52a-52e and 56a-56d are configured as disc magnets and can be made, for example, of samarium cobalt (SmCo), such as Sm₂Co₁₇ which can work up to 300°C to 350°C. Such magnets are particularly useful if the instrument 10 is not disposable, but is designed to be cleaned in an autoclave (at 121°C to 132°C). Permanent magnets such as neodymium iron boron (NdFeB) magnets may be used since they are the strongest magnets. Such strongest magnets are useful if the instrument 10 is to be scaled down to a very small diameter, for example 3 mm diameter. However, NdFeB magnets work normally up to 80°C although they can also work up to 120°C to 150°C with slightly reduced performance. Ceramic and other permanent magnets may be also considered depending on application requirements.

The attractive magnetic forces acting between the magnets 52a-52e and 56a-56d are the sole forces for closing and providing the grasping forces with the working element arrangement 28.

The hinge 42 and the hinge 44 are each configured as two-pin joints having pins 64, 66 (hinge 42) and 68, 70 (hinge 44).

The pin 64 serves as a conventional hinge joint in single-axis rotation of the segment 36 relative to the shaft 12, while the pin 66 travels together with the segment 36 in a curved opening 67 which limits the opening angle α (Fig. 3B), which can be, for example, in a range of 20° to 50°, for example 30°.

The arrangement of the pins 68, 70 of the hinge 44 is configured in a similar way, wherein the pin 70 travels together with the segment 34 in a curved opening 71. The hinges 42 and 44 are configured such that the segment 34 can be parallel to the working element 32 when the segment 34 is moved away from the working element 32, as shown in Fig. 3B. This is mainly due to a larger attractive magnetic force than repulsive force acted on the segment 34 at the opening initial stage, which forces the pin 70 travel toward the bottom (lower) end of the curved opening 71. The segment 34 remains parallel to the working element 32 in all positions from the fully open state to the closed state of the working element arrangement 28.

The segment 34 further has a grasping surface 72, and the working element 32 has a grasping surface 74 which, for example, have corrugated structures. However, differently from conventional grasping instruments, the structures of the grasping surfaces 72 and 74 may be blunt rather than sharp in order to reduce trauma to the tissue when being grasped, and nevertheless provide sufficient grip, also due to the parallel orientation of the segment 34 with respect to the working element 32.

The segment 34 forms the grasping segment of the working element 30 in cooperation with the grasping surface 74 of the working element 32. A stronger attractive force in the region of the segment 34 for closing than repulsive force for opening also facilitates the segment 34 opened in a parallel-like form.

In a variation of the example according to Fig. 3A and 3B, the displacement direction of the magnets 52a-52e for opening the working element arrangement 28 can be opposite to the arrow 58 in Fig. 3B. To this end, the magnet 52c is relocated from the center to the proximal end of the housing 60 (proximally of magnet 52e), and the displacement direction is changed and now is from the proximal to the distal direction, i.e. the rod 62 is pushed forward to open the working element arrangement 28 and pulled in proximal direction to close the working element arrangement 28. It is to be understood that in this variation the housing 60 has sufficient space on the distal side in the working element 32 to be pushed in distal direction for opening the working element arrangement 28. The push/pull rod can be connected with the movable grip part 18 as described before. However, the present variation is particularly advantageous, when a piezo- or linear motor is used at the proximal end of the instrument 10 to actuate the displacement of the housing 60. Further alternatively, a small diameter cylinder-shaped piezo-stack actuator or other small linear actuator may be integrated or assembled at the distal end of the shaft 12 of the instrument 10 to displace the housing 60 via a short push/pull rod.

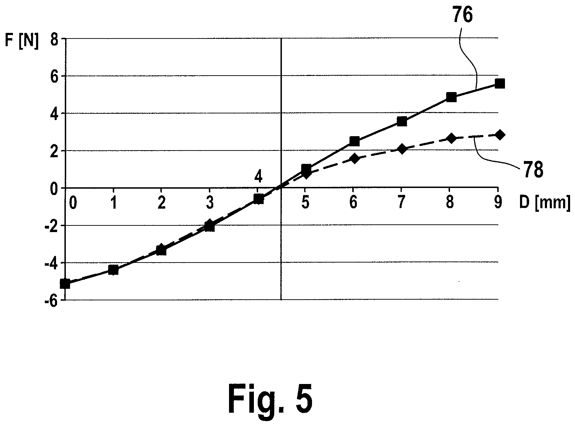

Fig. 5 shows, as an example for the example in Fig. 3A-B, the magnetic force F in Newton as a function of the position or displacement D of the magnets 52a-52ein millimeters (mm). A negative value of the force F means an attractive force, and a positive value of F means a repulsive force. A curve 76 shows the magnetic force acting between the segment 36 and the working element 32, while a curve 78 shows the force F acting between the segment 34 and the working element 32. The design of the working element arrangement 28 including the magnet arrangement 50 can be such that the magnetic attractive force is high for the segment 34 when the magnets 52a-52e are paired with the magnets 56a-56d as shown in Fig. 3A, while the magnetic repulsive force is higher for the segment 36 than for the segment 34 when the magnets 52a-52e are paired with the magnets 56a-56d as shown in Fig. 3B. Thus, the main function of the segment 36 is for opening the working element arrangement, while the main function of the segment 34 is for closing and grasping.

Fig. 4 shows a distal portion of the medical instrument 10 in Fig. 1A introduced through a body surface 80 for grasping tissue 82. The tissue 82 is grasped by the working element arrangement 28 between the segment 34 and the working element 32, which are parallel to one another, solely by magnetic attractive forces acting between the working element 30 and the working element 32. There is no risk of an excessive force or excessive pressure by using such a magnetic grasping instrument since the grasping force cannot exceed the maximum magnetic attraction force determined by the magnetic properties of the magnets 52a-52e and 56a-56d of the magnet arrangement 50 in the state as shown in Fig. 3A. The working element arrangement 28 forms a parallel-like grasper.

The maximum magnetic attractive force for closing, and thus, grasping tissue between the working elements 30 and 32 can be about 3 N to 6 N which is sufficient to grasp tissue for retraction purposes without causing any trauma to the grasped tissue. As already mentioned before, the maximum magnetic attractive force depends on the magnetic strength of the magnets used and/or the number of magnets used in the magnet arrangement 50.

While in the example described before and shown in Figs. 3A and 3B the magnet arrangement 50 has five magnets on the working element 32 and four magnets on the working element 30, the number of magnets of the magnet array or magnet arrangement 50 can differ therefrom. For example, the working element 32 can also have less or more than five magnets, for example eight magnets, and the working element 30 can have less or more than four magnets, for example seven magnets, four of which are arranged on the segment 34 and three of which are arranged on the segment 36.

Further, as shown in the example according to Figs. 3A and 3B, the magnets 52a-52e and 56a-56d are embedded within the bodies of the working elements 30, 32, but they can also be exposed to the mutually facing surfaces of the working elements 30 and 32. Preferably, the magnets 52a-52e are embedded within the body of the working element 32 since these magnets need to be slidably moved.

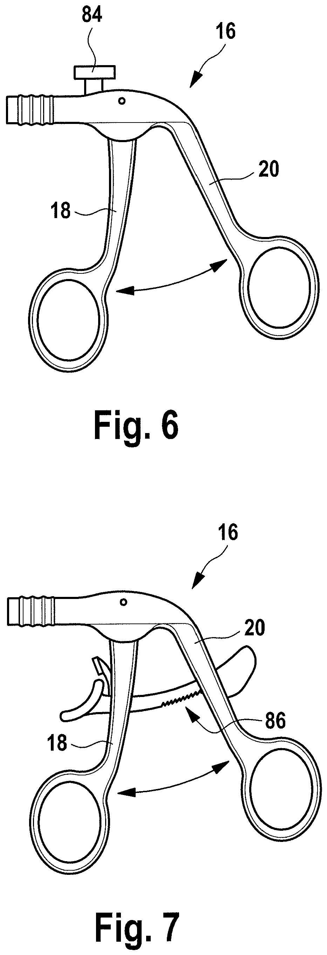

In case that actuation of the magnets 52a-52e is accomplished by an actuation of the movable grip part 18 of the handle 16, a locking mechanism can be used to fix an adjusted position of the displaceable magnets 52a-52eby locking the push/pull rod 62. For example, as shown in Fig. 6, a locking knob 84 arranged at the handle 16 can be used for locking the push/pull rod 62 in any position. Such a locking knob is described in the article: Frank, Cuschieri: Prehensile atraumatic grasper with intuitive ergonomics, surgical endoscopy 11 (1997): p. 1036-1039. Alternatively, a ratchet mechanism 86 arranged at the handle 16 as shown in Fig. 7 can be used. The ratchet mechanism 86 is configured to fix the movable grip part 18 at the stationary grip part 20 in different angular positions with respect to the stationary grip part 20, which is possible in a precise manner. Thus, when the movable grip part 18 is latched in a specific angular position, the push/pull rod 62 and thus the magnets 52a-52e are fixed in a specific longitudinal position. The ratchet mechanism 86 preferably is a disengageable ratchet mechanism.

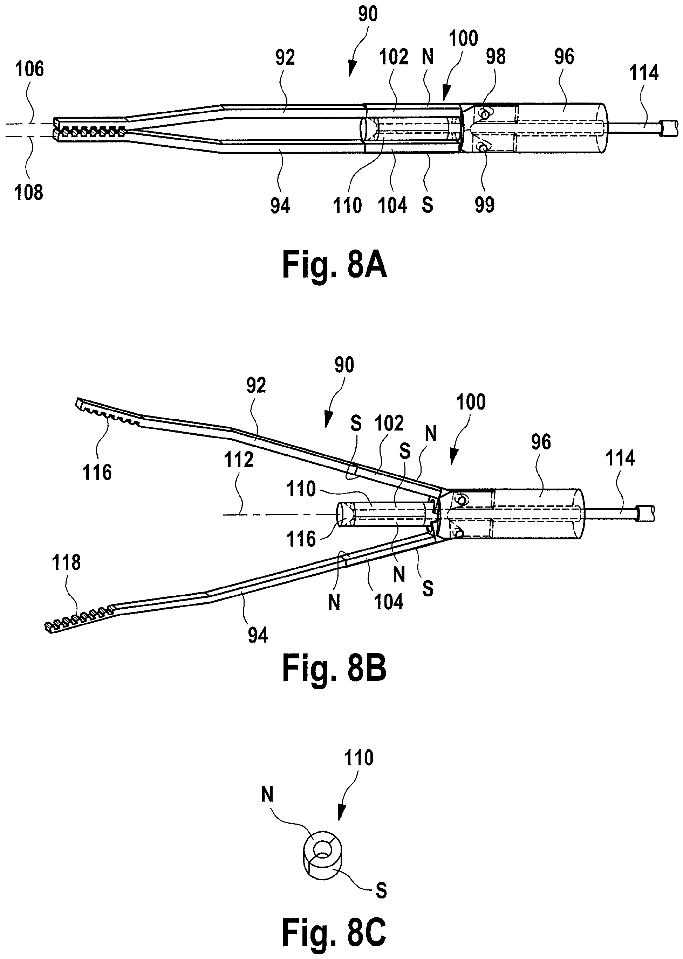

Figs. 8A and 8B show an embodiment of a working element arrangement 90 for grasping tissue.

Fig. 8A shows the working element arrangement 90 in a closed state, and Fig. 8B shows the working element arrangement 90 in an open state.

The working element arrangement 90 has two working elements 92, 94 configured as elongated fingers. Both working elements 92, 94 are movable, here pivotable, with respect to a shaft connector 96 forming a removable distal portion of the shaft of a medical instrument.

The working element 92 is pivotable about a hinge 98 which is shown as a one-pin joint, but a hinge like the hinge 42 in the previous example can be used in order to limit (or pre-set) a maximal opening angle of the working element 92. The other working element 94 is pivotable as well and connected to the shaft connector 96 via a further hinge 99 also shown as a one-pin joint, but a similar hinge as hinge 42 of the previous example can be used.

A magnet arrangement 100 is coupled to the working element arrangement 90 for opening and closing the working element arrangement 90 via magnetic repulsive and attractive forces, respectively.

The magnet arrangement 100 has a magnet 102 arranged on the working element 92, and a magnet 104 arranged on the working element 94.

The magnets 102 and 104 are configured as rectangle magnets or strip magnets, for example. The magnets 102 and 104 each have a magnetic polarity which is perpendicular to a longitudinal axis 106 and 108 of the working elements 92 and 94, respectively. In Fig. 8A, only the magnetic north pole N of the magnet 102 and the magnetic south pole of the magnet 104 are shown.

The magnetic polarities of the magnets 102 and 104 are parallel with respect to one another. Further, the magnetic polarities of the magnets 102 and 104 are perpendicular to the longitudinal axes 106 and 108 and parallel to the closing and opening direction of the working element arrangement 90.

The magnet arrangement 100 has another magnet 110 arranged between the working elements 92 and 94. The magnet 110 is rotatable about a rotation axis 112 which coincides with the longitudinal axis of the shaft connector 96 and, thus, of the shaft of the instrument. Fig. 8C shows the magnet 110 in isolation. The magnet 110 is configured as a ring magnet. It can, however, be a tube magnet, or a cylinder magnet, or a rod magnet. The magnet 110 has a polarity which is perpendicular (diametrical) with respect to the rotation axis 112.

The magnet 110 is connected to a rotatable actuating rod 114 which can be coupled, at the proximal end of the instrument, with an actuating wheel or the like. Also, a motor for rotating the actuating rod 114 can be provided.

Depending on the rotational position of the magnet 110, the magnetic north pole N of the magnet 110 can face the magnetic south pole of the magnet 102 and the magnetic south pole S of the magnet 110 then faces the magnetic north pole of the magnet 104. In this case, a magnetic attractive force acts between the magnets 102, 104 and the magnet 110, respectively, thus the working elements 92 and 94 are closed as shown in Fig. 8A.

When the magnet 110 is rotated such that the magnetic north pole N of the magnet 110 faces the magnetic north pole of the magnet 104 and the magnetic south pole S of the magnet 110 faces the magnetic south pole of the magnet 102, magnetic repulsive forces act between the magnets 102, 104 and 110 so as to open the working elements 92 and 94 as shown in Fig. 8B.

The magnetic attractive force between the magnets 102, 104 and 110 can be varied due to the degree of rotation of the magnet 110, thus a range of magnitudes of the magnetic coupling force can be obtained for tissue grasping.

A lamp, for example a LED or a LED array 116 can be advantageously arranged distally of the magnet 110, in order to light the surgical site. Alternatively or in addition, an imaging module for imaging purposes can be arranged distally of the magnet 110. Preferably, the imaging module is stationary while the magnet 110 can be rotated.

The configuration of the working element arrangement 90 is very similar to "chop-sticks" which has benefits in precision tissue grasping, for example in microsurgery or other surgical applications.

The working elements 92, 94 each have a grasping surface 116 and 118, respectively with, for example, a surface profile as shown. The surface profile, again only has blunt structures in order to avoid trauma to the grasped tissue.

The magnetic attractive and repulsive forces are the sole forces for opening and closing the working element arrangement 90.

In the embodiment shown, the magnet arrangement 100 is arranged in a proximal non-grasping area of the working elements 92 and 94 which are configured as elongate fingers, while the distal grasping area formed by the grasping surfaces 116 and 118 are spaced apart from the magnet arrangement 100.

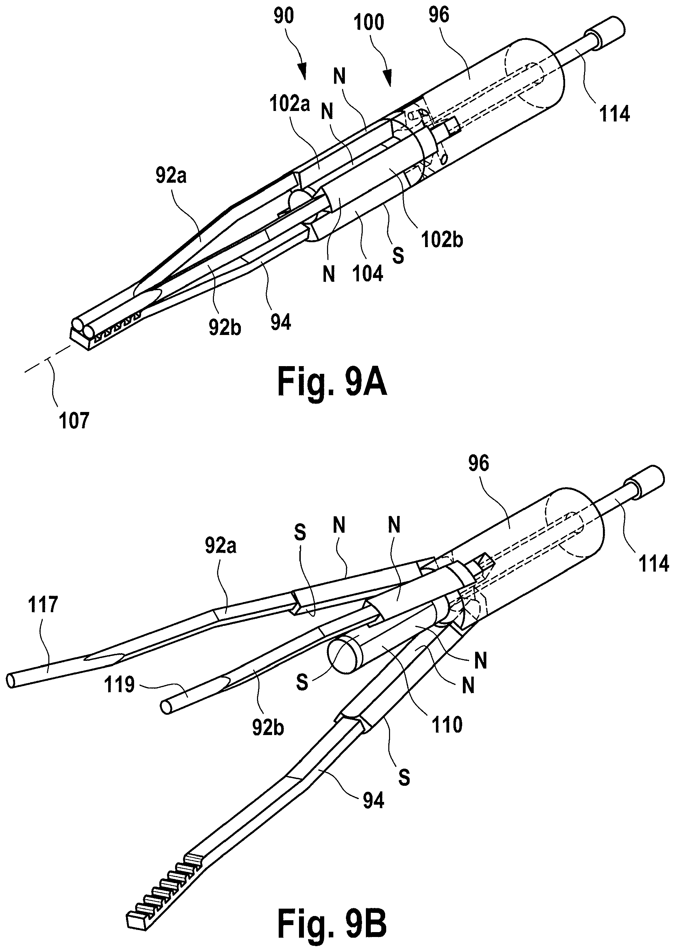

Figs. 9A and 9B show a modification of the embodiment in Figs. 8A and 8B. Those elements of the embodiment in Figs. 9A and 9B which are comparable with elements of the embodiment in Figs. 8A and 8B are provided with the same reference numerals as in Fig. 8A and 8B.

Differently from the previous embodiment, the working element arrangement 90 has three working elements 92a, 92b, 94. This configuration provides human hand-like prehensile grasping of tissue. The magnet arrangement 100 has a magnet 102a arranged on the working element 92a, a magnet 102b arranged on the working element 92b and a magnet 104 arranged on the working element 94. The magnetic polarities of the magnets 102a and 102b lie in a plane perpendicular to the longitudinal axis 107 of the working element arrangement 90. The polarities of the magnets 102a and 102b can be slightly inclined with respect to each other and with respect to the polarity of magnet 104. Such a slight angularity between these polarities can still be considered as parallel polarities. Another magnet 110 configured as a ring magnet like the magnet 110 in Fig. 8C is arranged between the working elements 92a, 92b and 94. The working elements 92a, 92b and the working element 94 are circumferentially distributed about the magnet 110.

Again, the magnet 110 is rotatable about the longitudinal axis 107 of the working element arrangement 90 through a rotatable actuating rod 114 as in the previous embodiment.

Thus, by rotating the magnet 110 about the longitudinal axis 107 as the rotation axis, the working element arrangement 90 can be opened and closed solely through magnetic forces. A range of magnitudes of the magnetic coupling force can be obtained by a rotation of the magnet 110 due to the degree of rotation of the magnet 110.

The working elements 92a and 92b each have a grasping area 117 and 119, respectively which do not have profiled surfaces in this example, but are more or less smooth. In the embodiment shown, the grasping areas 117 and 119 of the working elements 92a and 92b are configured as cylindrical rods. It is to be understood that the grasping areas 117 and 119 can also be configured differently, for example with rectangular cross-sections and/or with a surface profiling. For example, grasping surfaces 117, 119 can have profiled surfaces like grasping surface 116 in Fig. 8B.

In the embodiments of Fig. 8A, 8B and 9A, 9B, the number of magnets on the working elements 92, 94; 92a, 92b, 94 and between them can be higher than one, in particular an array of magnets can be used on the working elements and a magnet array between the working elements.

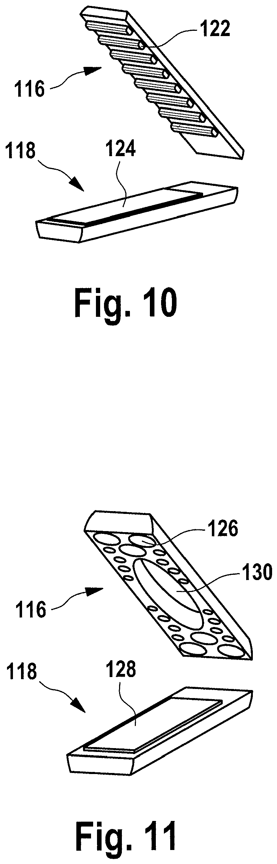

Figs. 10 through 12 show embodiments of a magnetic surface profiling of working elements, which, for example, can be used in the working elements 92 and 94 as the grasping surfaces 116 and 118 in Figs. 8A and 8B.

Fig. 10 shows an embodiment where the grasping surface 116 has an array of magnets 122 configured in form of magnetic strips. The magnetic strips 122 can replace the profile structures of the grasping surface 116 in Figs. 8A and 8B.

The grasping surface 118 has a single magnet 124 in form of a magnetic plate (or a rectangular magnet). The magnetic polarities of the magnets 122 and of the magnet 124 are such that the magnets 122 and the magnet 124 attract each other. Alternatively, the strips 122 can be ferromagnetic stainless steel which can be attracted to the permanent magnet 124.

Fig. 11 shows another embodiment, in which the grasping surface 116 has an array of small disk magnets 126 embedded in the grasping surface 116. As shown, the magnets 126 can have different sizes with respect to each other and are distributed over the grasping surface 116.

The grasping surface 118 again has a single magnet 128 configured as a magnetic plate (or a rectangular magnet). Further, the grasping surface 116 has a fenestration 130 for enhancing the tissue retention capability.

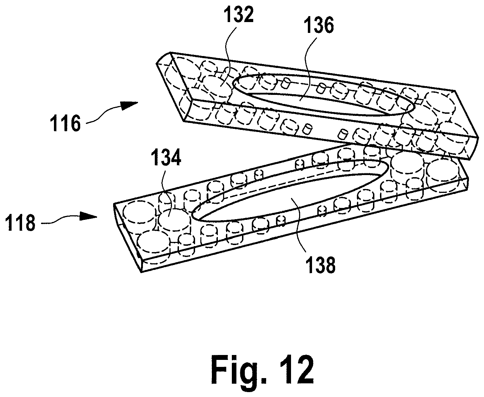

A modification of the embodiment in Fig. 11 is shown in Fig. 12, wherein both the grasping surface 116 and the grasping surface 118 each have an array of disk magnets 132 and 134 embedded therein. Each of the magnets 132 is paired with one of the magnets 134, with the magnetic polarities oriented such that the magnets 132 and the magnets 134 attract each other. In the embodiment shown, the distribution of the magnets 132 in terms of size and position is identical with the distribution of the magnets 134, thus forming a symmetrical arrangement of the magnets 132 and 134. Further, both grasping surfaces 116 and 118 have a fenestration 136 and 138, respectively.

The magnetic surface profiling according to Figs. 10 through 12 can replace any conventional surface profiles, which reduces trauma to tissue grasped while ensuring a good retention of the tissue between the grasping surfaces 116 and 118.

## Claims

1. A medical instrument for grasping tissue, comprising a shaft, a working element arrangement (90) arranged at a distal end of the shaft and having a first working element (92; 92a) and a second working element (94) configured to cooperate with one another for grasping tissue, wherein at least the first working element (92; 92a) is movable, so that the working element arrangement (90) can be closed and opened, further comprising a magnet arrangement (100) coupled to the working element arrangement (90) for closing and opening the working element arrangement (90) via attracting and repulsive magnetic forces, respectively, wherein the first and second working elements (92; 92a, 94) are movable, wherein the magnet arrangement (90) has at least three magnets (102; 102a, 104, 110) wherein at least one first magnet (102; 102a) is arranged on the first working element (92; 92a), at least one second magnet (104) is arranged on the at least one second working element (94), the first and second magnets (102; 102a, 104) having magnetic polarities perpendicular to a longitudinal axis (106, 108) of the first and second working elements (92; 92a, 94), respectively, **characterized in that** at least one third magnet (110) is arranged between the first and second working elements (92; 92a, 94), the third magnet (110) being rotatable about a rotation axis (112).

2. The medical instrument of claim 1, **characterized in that** the first and second magnets (102; 102a, 104) have parallel polarities with respect to one another and parallel to the closing and opening movement of the first and second working elements (92; 92a, 94).

3. The medical instrument of claim 1 or 2, **characterized in that** the rotation axis (112) is parallel to a longitudinal axis of the shaft, and the third magnet (110) has a magnetic polarity perpendicular to the rotation axis (112).

4. The medical instrument of any one of claims 1 through 3, **characterized in that** the third magnet (110) is connected to a rotatable actuating rod (114).

5. The medical instrument of any one of claims 1 through 4, **characterized in that** the first and second working elements (92; 92a, 94) are configured as elongate fingers each having a distal grasping area (116; 117, 118), and wherein the first and second magnets (102; 102a, 104) are arranged in a proximal non-grasping area of the elongate fingers.

6. The medical instrument of any one of claims 1 through 4, **characterized by** at least one third working element (92b) having a further magnet (102b), wherein the at least three working elements (92a, 92b, 94) are circumferentially distributed about the third magnet (110).

7. The medical instrument of any one of claims 1 through 6, **characterized in that** the magnetic attractive force is the sole force for closing the working element arrangement (28; 90), and/or the magnetic repulsive force is the sole force for opening the working element arrangement (28; 90).

8. The medical instrument of any one of claims 1 through 7, **characterized in that** the first working element (92) and the second working element (94) each has a grasping surface (116, 118) for grasping tissue, one (116) of the grasping surfaces (116, 118) having an array of first further magnets (122; 126, 132), and the at least one other grasping surface (118) has at least one second further magnet (124; 128) or an array of second further magnets (134) for attractive interaction with the first further magnets (122; 126; 132).

## Patentansprüche

1. Medizinisches Instrument zum Fassen von Gewebe, mit einem Schaft, einer Arbeitselementanordnung (90), die an einem distalen Ende des Schaftes angeordnet ist und ein erstes Arbeitselement (92; 92a) und ein zweites Arbeitselement (94) aufweist, die dazu ausgelegt sind, miteinander zum Fassen von Gewebe zusammenzuwirken, wobei zumindest das erste Arbeitselement (92; 92a) beweglich ist, so dass die Arbeitselementanordnung (90) geschlossen und geöffnet werden kann, weiterhin mit einer Magnetanordnung (100), die mit der Arbeitselementanordnung (90) zum Schließen und Öffnen der Arbeitselementanordnung (90) mittels anziehender beziehungsweise abstoßender Magnetkräfte gekoppelt ist, wobei das erste und das zweite Arbeitselement (92; 92a, 94) beweglich sind, wobei die Magnetanordnung (90) zumindest drei Magnete (102; 102a, 104) aufweist, wobei zumindest ein erster Magnet (102; 102a) an dem ersten Arbeitselement (92; 92a) angeordnet ist, zumindest ein zweiter Magnet (104) an dem zumindest einen zweiten Arbeitselement (94) angeordnet ist, wobei der erste und der zweite Magnet (102; 102a, 104) magnetische Polaritäten senkrecht zu einer Längsachse (106, 108) des ersten beziehungswiese zweiten Arbeitselements (92; 92a, 94) aufweisen, **dadurch gekennzeichnet, dass** zumindest ein dritter Magnet (110) zwischen dem ersten und zweiten Arbeitselement (92; 92a, 94) angeordnet ist, wobei der dritte Magnet (110) um eine Drehachse (112) drehbar ist.

2. Medizinisches Instrument nach Anspruch 1, **dadurch gekennzeichnet, dass** der erste und zweite Magnet (102; 102a, 194) parallele Polaritäten bezüglich zueinander und parallel zur Schließ- und Öffnungsbewegung des ersten und zweiten Arbeitselementes (92; 92a, 94) aufweisen.

3. Medizinisches Instrument nach Anspruch 1 oder 2, **dadurch gekennzeichnet, dass** die Drehachse (112) parallel zu einer Längsachse des Schaftes verläuft, und der dritte Magnet (110) eine magnetische Polarität senkrecht zu der Drehachse (112) aufweist.

4. Medizinisches Instrument nach einem der Ansprüche 1 bis 3, **dadurch gekennzeichnet, dass** der dritte Magnet (110) mit einem drehbaren Betätigungsstab (114) verbunden ist.

5. Medizinisches Instrument nach einem der Ansprüche 1 bis 4, **dadurch gekennzeichnet, dass** das erste und zweite Arbeitselement (92; 92a, 94) als längliche Finger ausgebildet sind, von denen jeder einen distalen Fassbereich (116; 117, 118) aufweist, und wobei der erste und zweite Magnet (102; 102a, 104) in einem proximalen Nicht-Fassbereich der länglichen Finger angeordnet sind.

6. Medizinisches Instrument nach einem der Ansprüche 1 bis 4, **gekennzeichnet durch** zumindest ein drittes Arbeitselement (92b), das einen weiteren Magneten (102b) aufweist, wobei die zumindest drei Arbeitselemente (92a, 92b, 94) umfänglich um den dritten Magneten (110) verteilt sind.

7. Medizinisches Instrument nach einem der Ansprüche 1 bis 6, **dadurch gekennzeichnet, dass** die magnetische Anziehungskraft die einzige Kraft zum Schließen der Arbeitselementanordnung (98; 90) ist, und/oder die magnetische Abstoßungskraft die einzige Kraft zum Öffnen der Arbeitselementanordnung (28; 90) ist.

8. Medizinisches Instrument nach einem der Ansprüche 1 bis 7, **dadurch gekennzeichnet, dass** das erste Arbeitselement (92) und das zweite Arbeitselement (94) jeweils eine Fassfläche (116, 118) zum Fassen von Gewebe aufweisen, wobei eine (116) der Fassflächen (116, 118) ein Array von ersten weiteren Magneten (122; 126, 132) aufweist, und die zumindest eine andere Fassfläche (118) zumindest einen zweiten weiteren Magneten (124; 128) oder ein Array von zweiten weiteren Magneten (134) zur anziehenden Wechselwirkung mit den ersten weiteren Magneten (122; 126; 132) aufweist.

## Revendications

1. Instrument médical pour saisir un tissu, comprenant un arbre, un agencement d'éléments de travail (90) agencé au niveau d'une extrémité distale de l'arbre et ayant un premier élément de travail (92 ; 92a) et un deuxième élément de travail (94) configurés pour coopérer l'un avec l'autre pour saisir un tissu, dans lequel au moins le premier élément de travail (92 ; 92a) est mobile, de sorte que l'agencement d'éléments de travail (90) puisse être fermé et ouvert, comprenant en outre un agencement d'aimants (100) couplé à l'agencement d'éléments de travail (90) pour assurer la fermeture et l'ouverture de l'agencement d'éléments de travail (90) par l'intermédiaire de forces magnétiques d'attraction et de répulsion, respectivement, dans lequel les premier et deuxième éléments de travail (92 ; 92a, 94) sont mobiles, dans lequel l'agencement d'aimants (90) a au moins trois aimants (102 ; 102a, 104, 110) dans lequel au moins un premier aimant (102 ; 102a) est agencé sur le premier élément de travail (92 ; 92a), au moins un deuxième aimant (104) est agencé sur l'au moins un deuxième élément de travail (94), les premier et deuxième aimants (102 ; 102a, 104) ayant des polarités magnétiques perpendiculaires à un axe longitudinal (106, 108) des premier et deuxième éléments de travail (92 ; 92a, 94), respectivement, **caractérisé en ce qu'**au moins un troisième aimant (110) est agencé entre les premier et deuxième éléments de travail (92 ; 92a, 94), le troisième aimant (110) pouvant tourner autour d'un axe de rotation (112).

2. Instrument médical de la revendication 1, **caractérisé en ce que** les premier et deuxième aimants (102 ; 102a, 104) ont des polarités parallèles l'un par rapport à l'autre et parallèles au mouvement de fermeture et d'ouverture des premier et deuxième éléments de travail (92 ; 92a, 94).

3. Instrument médical de la revendication 1 ou 2, **caractérisé en ce que** l'axe de rotation (112) est parallèle à un axe longitudinal de l'arbre, et le troisième aimant (110) a une polarité magnétique perpendiculaire à l'axe de rotation (112).

4. Instrument médical de l'une quelconque des revendications 1 à 3, **caractérisé en ce que** le troisième aimant (110) est relié à une tige d'actionnement rotative (114).

5. Instrument médical de l'une quelconque des revendications 1 à 4, **caractérisé en ce que** les premier et deuxième éléments de travail (92 ; 92a, 94) sont configurés sous forme de doigts allongés ayant chacun une zone de saisie distale (116 ; 117, 118), et dans lequel les premier et deuxième aimants (102 ; 102a, 104) sont agencés dans une zone de non-saisie proximale des doigts allongés.

6. Instrument médical de l'une quelconque des revendications 1 à 4, **caractérisé par** au moins un troisième élément de travail (92b) ayant un aimant supplémentaire (102b), dans lequel les au moins trois éléments de travail (92a, 92b, 94) sont répartis de manière circonférentielle autour du troisième aimant (110).

7. Instrument médical de l'une quelconque des revendications 1 à 6, **caractérisé en ce que** la force d'attraction magnétique est la seule force pour la fermeture de l'agencement d'éléments de travail (28 ; 90), et/ou la force de répulsion magnétique est la seule force pour l'ouverture de l'agencement d'éléments de travail (28 ; 90).

8. Instrument médical de l'une quelconque des revendications 1 à 7, **caractérisé en ce que** le premier élément de travail (92) et le deuxième élément de travail (94) ont chacun une surface de saisie (116, 118) pour saisir un tissu, l'une (116) des surfaces de saisie (116, 118) ayant un réseau de premiers aimants supplémentaires (122 ; 126, 132), et l'au moins une autre surface de saisie (118) a au moins un deuxième aimant supplémentaire (124 ; 128) ou un réseau de deuxièmes aimants supplémentaires (134) pour une interaction attractive avec les premiers aimants supplémentaires (122 ; 126 ; 132).
